# EUROPEAN PATENT SPECIFICATION

(11) **EP 3 609 536 B1**
(45) Date of publication and mention of the grant of the patent: **09.03.2022**
(21) Application number: 17889643.7
(22) Date of filing: 05.01.2017
(51) Int. Cl.: A61K 39/395, C07K 16/28, A61P 35/00

(54) **HUMANIZED ANTI-CD19 ANTIBODY AND USE THEREOF WITH CHIMERIC ANTIGEN RECEPTOR**
HUMANISIERTER ANTI-CD19-ANTIKÖRPER UND VERWENDUNG DAVON MIT CHIMÄREM ANTIGENREZEPTOR
ANTICORPS ANTI-CD19 HUMANISÉ ET SON UTILISATION AVEC UN RÉCEPTEUR D'ANTIGÈNE CHIMÈRE

(43) Date of publication of application: 19.02.2020
(60) Divisional application: 22152822.7
(73) Proprietor: Innovative Cellular Therapeutics Holdings, Ltd., Grand Cayman, KYI - 1104 (KY)
(72) Inventor: WU, Zhao, Shanghai 201203 (CN); LIU, Zhigang, Beijing 100071 (CN); XIAO, Lei, Shanghai 201203 (CN); PU, Chengfei, Shanghai 201203 (CN); CAO, Zhiyuan, Shanghai 201203 (CN)
(74) Representative: Abel & Imray LLP
(86) International application number: PCT/CN2017/070208
(87) International publication number: WO 2018/126369

(56) References cited:
- WO-A1-2012/010562
- WO-A1-2014/153270
- WO-A1-2016/033570
- WO-A2-2005/012493
- WO-A2-2007/002223
- WO-A2-2010/095031
- WO-A2-2010/095031

## Description

### TECHNICAL FIELD

This disclosure relates to biotechnology. More specifically, the disclosure relates to humanized antibodies and uses thereof.

### BACKGROUND

CD19 is a hallmark of B-cells since CD19 is expressed on the surface of human B cells including pre-B cells, immature B cells, mature B cells and malignant B cells. CD19 has been used to diagnose cancers that arise from B cell (e.g., B-cell lymphomas). Further, some drugs associated with anti-CD 19 may direct treatment specifically towards B-cell cancers. For example, CD19 monoclonal antibody may be used for the treatment of B-cell related diseases.

Monoclonal antibodies are typically made entirely from mouse cells. One problem with this is that the human immune system may see these antibodies as foreign and may mount a response against them. In the short term, this can sometimes cause an immune response. In the long term, this may cause the antibodies to only work the first time(s) they are given. After the first time, the human immune system may destroy these antibodies before the desired treatment takes place.

WO2016/033570 (D1) discloses CD19 binding molecules, including anti-CD19 antibodies, including antibody fragments such as single-chain fragments, and chimeric receptors including the antibodies, such as chimeric antigen receptors (CARs). Among the antibodies are human antibodies, including those that compete for binding to CD19 with reference antibodies, such as murine antibodies. In some embodiments of WO2016/033570, the antibodies display similar functional properties to the reference antibodies, such as comparable binding affinities and/or competitive inhibition properties. WO2016/033570 also discloses genetically engineered cells expressing the chimeric receptors, and uses of the binding molecules and cells adoptive cell therapy.

WO2010/095031 (D2) discloses humanized antibodies or fragments thereof that bind to human CD19. More specifically, WO2010/095031 discloses a humanized antibody or fragment thereof that binds to human CD19 comprising a heavy chain CDR1 comprising the amino acid sequence of SEQ ID NO: 27 of WO2010/095031, and/or a heavy chain CDR2 comprising the amino acid sequence of SEQ ID NO: 28 of WO2010/095031, and/or a heavy chain CDR3 comprising the amino acid sequence of SEQ ID NO: 29 of WO2010/095031; and/or comprising a light chain CDR1 comprising the amino acid sequence of SEQ ID NO: 30 of WO2010/095031, and/or a light chain CDR2 comprising the amino acid sequence of SEQ ID NO: 31 of WO2010/095031 and/or a light chain CDR3 comprising the amino acid sequence of SEQ ID NO: 32 of WO2010/095031.

### SUMMARY

The present invention provides a human T cell according to claim 1.

The present invention further provides a pharmaceutical composition according to claim 5. The present invention also provides the human T cell of claim 1, or a pharmaceutical composition of claim 5, for use in the treatment of cancer.

The embodiments herein find utility in a method for treating a subject having a CD19 positive tumor. The method may include administering a modified cell to the subject having the CD19 positive tumor. The modified cell includes an intracellular domain and an antigen recognition domain that includes the humanized antibody.

Embodiments relate to a human T cell including a nucleic acid sequence encoding a CAR. The CAR includes a CD19 antigen binding domain including the amino acid sequence of SEQ ID NO:37. The CAR further includes a transmembrane domain, and an intracellular domain and a signaling domain of a costimulatory molecule.

In some embodiments, the intracellular domain may include a CD3-zeta signaling domain.

In embodiments, the antigen binding fragment is a scFv. The scFv includes the amino acid sequence of SEQ ID NO:37.

In some embodiments, the T cell comprises a vector that comprises the nucleic acid sequence. For example, the vector is a lentiviral vector.

In some embodiments, a pharmaceutical composition comprising the human T cell as described above.

### BRIEF DESCRIPTION OF THE DRAWINGS

The detailed description is described with reference to the accompanying figures.

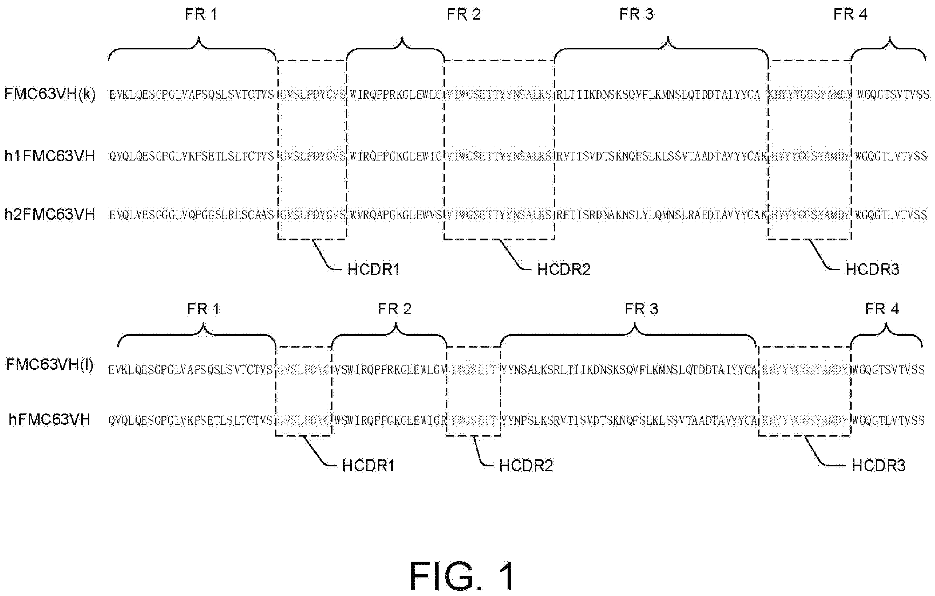

FIG. 1 illustrates a sequence alignment comparing the amino acid sequences of each variable domains of the heavy chain of FMC63VH (SEQ ID: 1), hFMC63VH (SEQ ID: 25), h1FMC63VH (SEQ ID: 26), and h2FMC63VH (SEQ ID: 27) according to Kabat or IMGT database definitions.
FIG. 2 illustrates a sequence alignment comparing the amino acid sequences of variable domains of the light chain of L2D8 (SEQ ID: 34), L2D12 (SEQ ID: 35), and L5F3 (SEQ ID: 36).
FIG. 3 shows the results of reducing SDS-PAGE (10%) analysis of the purified recombinant full-length FMC63 antibodies. M indicates protein molecular weight standards (170, 130, 100, 70, 55, 40, 35, 25, 15, 10kD); FMC63 indicates purified recombinant murine monoclonal antibody FMC63. The heavy chain is about 50kD, and the light chain is about 25KD.
FIG. 4 shows the results of reducing SDS-PAGE (15%) analysis of purified recombinant CD19ECD-His fusion protein. M indicates protein molecular weight standards (170, 130, 100, 70, 55, 40, 35, 25, 15, 10kD); "1" indicates purified recombinant CD19ECD-His; "2" indicates purified recombinant CD19ECD-His.
FIG. 5 illustrates results of ELISA analysis of recombinant murine antibody FMC63 in combination with different protein antigens. CD19-His: recombinant human CD19 extracellular domain with the His-tag fusion protein; IL17-His: recombinant human IL17 fusion protein with the His-tag; PCSK9-His: recombinant human PCSK9 with a His-tag fusion protein; BSA: bovine serum albumin protein.
FIG. 6 shows the results of reducing SDS-PAGE (15%) analysis of the purified recombinant four kinds FMC63 full-length antibodies. M indicates protein molecular weight standards (170, 130, 100, 70, 55, 40, 35, 25, 15, 10kD); "1" indicates purified recombinant chimeric FMC63; "2" indicates the purified recombinant hFMC63; "3" indicates purified recombinant h2FMC63. The heavy chain is about 50KD, and the light chain is about 25KD.

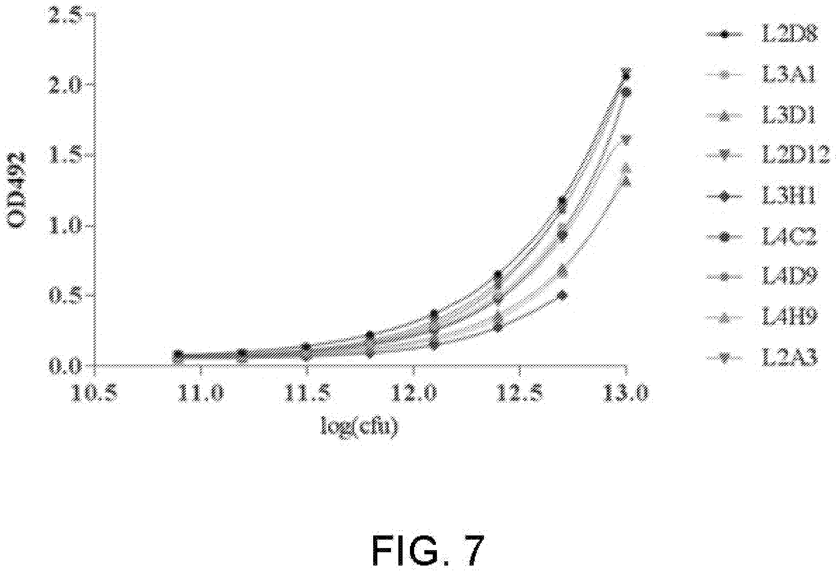

FIG. 7 illustrates a comparison between the relative affinities of different monoclonal antibodies measured using purified phage-Fab.

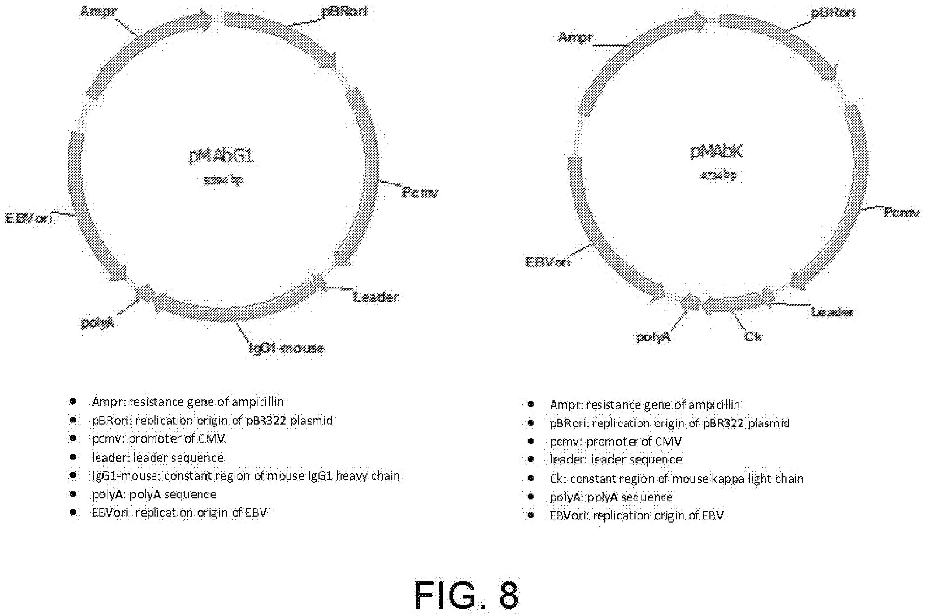

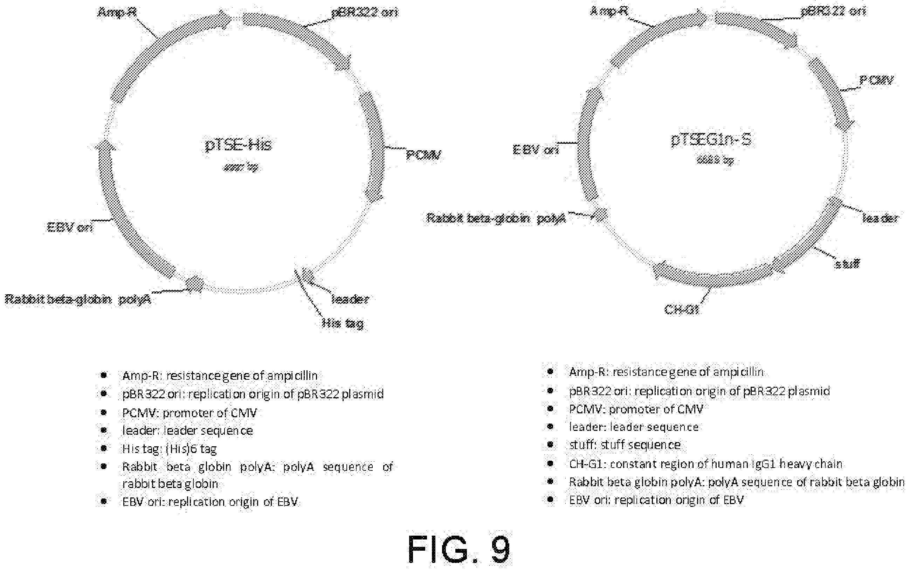

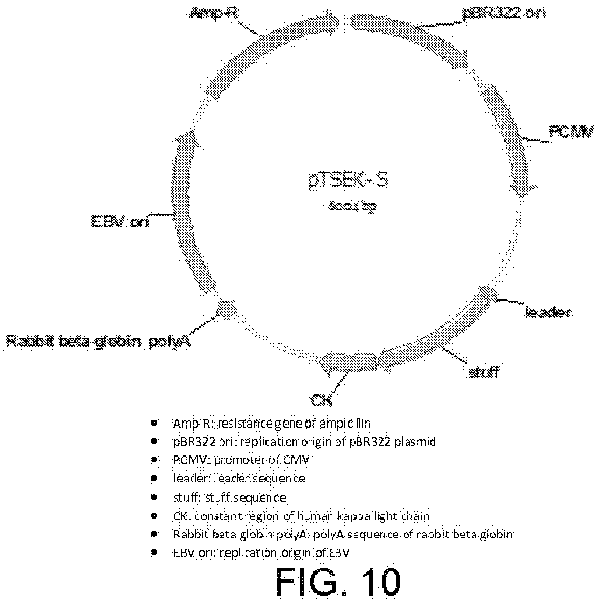

FIGS. 8-10 show the vectors for expression of one or more polynucleotides in host cells in accordance with embodiments herein.
FIG. 11 is a schematic diagram illustrating molecular structures of two CARs.

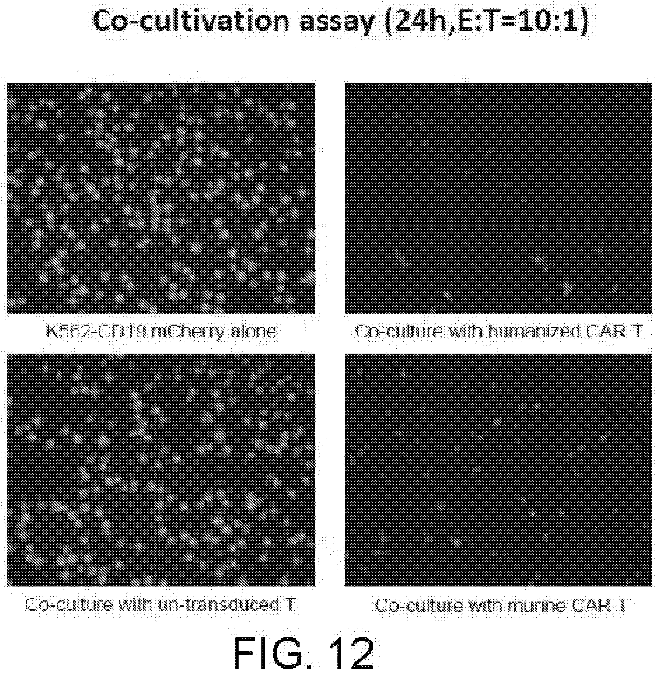

FIG. 12 show that T cells express two CARs provided in FIG. 11 killed CD19 positive cells when these T cells were co-cultured with CD19-positive cells.

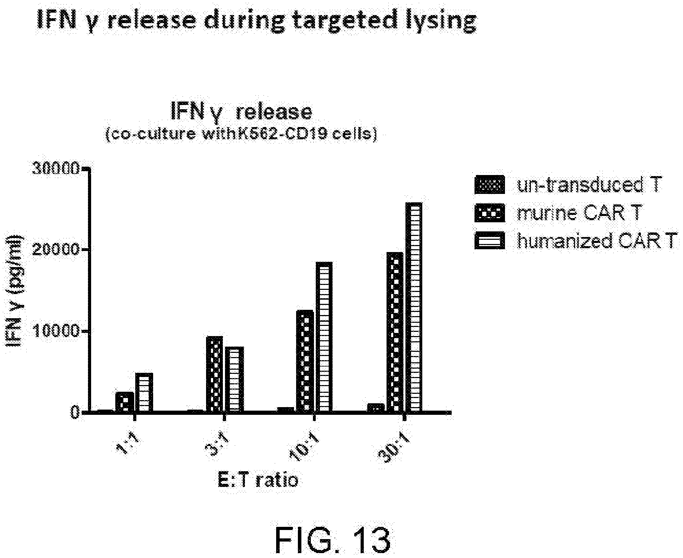

FIG. 13 illustrating comparison of amounts of IFN-gamma that are released from T cells expressing two CARs provided in FIG. 11, respectively.

### DETAILED DESCRIPTION

Unless defined otherwise, all technical and scientific terms used herein have the same meaning as commonly understood by those of ordinary skill in the art to which the present disclosure belongs. Although any methods and materials similar or equivalent to those described herein can be used in the practice or testing of the present disclosure, methods, and materials are described. For the purposes of the present disclosure, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

By "coding sequence" is meant any nucleic acid sequence that contributes to the code for the polypeptide product of a gene. By contrast, the term "non-coding sequence" refers to any nucleic acid sequence that does not contribute to the code for the polypeptide product of a gene.

Throughout this specification, unless the context requires otherwise, the words "comprise," "comprises" and "comprising" will be understood to imply the inclusion of a stated step or element or group of steps or elements but not the exclusion of any other step or element or group of steps or elements.

By "consisting of' is meant including, and limited to, whatever follows the phrase "consisting of." Thus, the phrase "consisting of' indicates that the listed elements are required or mandatory and that no other elements may be present.

By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but those other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

By "corresponds to" or "corresponding to" is meant (a) a polynucleotide having a nucleotide sequence that is substantially identical or complementary to all or a portion of a reference polynucleotide sequence or encoding an amino acid sequence identical to an amino acid sequence of a peptide or protein; or (b) a peptide or polypeptide having an amino acid sequence that is substantially identical to a sequence of amino acids in a reference peptide or protein.

As used herein, the terms "function" and "functional" and the like refer to a biological, binding, or therapeutic function.

By "gene" is meant a unit of inheritance that occupies a specific locus on a chromosome and consists of transcriptional and/or translational regulatory sequences and/or a coding region and/or non-translated sequences (i.e., introns, 5' and 3' untranslated sequences).

"Homology" refers to the percentage number of amino acids that are identical or constitute conservative substitutions. Homology may be determined using sequence comparison programs such as GAP (Deveraux et al., 1984, Nucleic Acids Research 12, 387-395). In this way, sequences of a similar or substantially different length to those cited herein could be compared by insertion of gaps into the alignment, such gaps being determined, for example, by the comparison algorithm used by GAP.

The term "host cell" includes an individual cell or cell culture which can be or has been a recipient of any recombinant vector(s) or isolated polynucleotide of the present disclosure. Host cells include progeny of a single host cell, and the progeny may not necessarily be completely identical (in morphology or in total DNA complement) to the original parent cell due to natural, accidental, or deliberate mutation and/or change. A host cell includes cells transfected or infected in vivo or in vitro with a recombinant vector or a polynucleotide of the present disclosure. A host cell which comprises a recombinant vector of the present disclosure is a recombinant host cell. For example, a cell that produces a humanized CD19 antibody of the present disclosure will include the bacterial and eukaryotic host cells into which nucleic acid encoding the antibodies have been introduced.

An "isolated" antibody is one which has been identified and separated and/or recovered from a component of its natural environment. Contaminant components of its natural environment are materials which would interfere with diagnostic or therapeutic uses for the antibody and may include enzymes, hormones, and other proteinaceous or nonproteinaceous solutes.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The recitation "polynucleotide" or "nucleic acid" as used herein designates mRNA, RNA, cRNA, rRNA, cDNA or DNA. The term typically refers to a polymeric form of nucleotides of at least 10 bases in length, either ribonucleotides or deoxynucleotides or a modified form of either type of nucleotide. The term includes single and double stranded forms of DNA and RNA.

The terms "polynucleotide variant" and "variant" and the like refer to polynucleotides displaying substantial sequence identity with a reference polynucleotide sequence or polynucleotides that hybridize to a reference sequence under stringent conditions that are defined hereinafter. These terms also encompass polynucleotides that are distinguished from a reference polynucleotide by the addition, deletion or substitution of at least one nucleotide. Accordingly, the terms "polynucleotide variant" and "variant" include polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide, or has increased activity in relation to the reference polynucleotide (i.e., optimized). Polynucleotide variants include, for example, polynucleotides having at least 50% (and at least 51% to at least 99% and all integer percentages in between, e.g., 90%, 95%, or 98%) sequence identity with a reference polynucleotide sequence described herein. The terms "polynucleotide variant" and "variant" also include naturally-occurring allelic variants and orthologs that encode these enzymes.

"Polypeptide," "polypeptide fragment," "peptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally occurring amino acid polymers. In certain aspects, polypeptides may include enzymatic polypeptides, or "enzymes," which typically catalyze (i.e., increase the rate of) various chemical reactions.

The recitation polypeptide "variant" refers to polypeptides that are distinguished from a reference polypeptide sequence by the addition, deletion or substitution of at least one amino acid residue. In certain embodiments, a polypeptide variant is distinguished from a reference polypeptide by one or more substitutions, which may be conservative or non-conservative. In certain embodiments, the polypeptide variant comprises conservative substitutions and, in this regard; it is well understood in the art that some amino acids may be changed to others with broadly similar properties without changing the nature of the activity of the polypeptide. Polypeptide variants also encompass polypeptides in which one or more amino acids have been added or deleted, or replaced with different amino acid residues.

The term "reference sequence" generally refers to a nucleic acid coding sequence, or amino acid sequence, to which another sequence is being compared. All polypeptide and polynucleotide sequences described herein are included as references sequences.

The recitations "sequence identity" or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 97%, 98%, 99% or 100% sequence identity to any of the reference sequences described herein (see, e.g., Sequence Listing), typically where the polypeptide variant maintains at least one biological activity of the reference polypeptide.

By "statistically significant," it is meant that the result was unlikely to have occurred by chance. Statistical significance can be determined by any method known in the art. Commonly used measures of significance include the p-value, which is the frequency or probability with which the observed event would occur if the null hypothesis were true. If the obtained p-value is smaller than the significance level, then the null hypothesis is rejected. In simple cases, the significance level is defined at a p-value of 0.05 or less.

"Substantially" or "essentially" means nearly totally or completely, for instance, 95%, 96%, 97%, 98%, 99% or greater of some given quantity.

"Treating" or "treatment" or "alleviation" refers to both therapeutic treatment and prophylactic or preventative measures, wherein the object is to prevent or slow down (lessen) the targeted pathologic condition or disorder. For example, a subject is successfully "treated" for a CD19 positive cancer or an autoimmune disease if, after receiving a therapeutic amount of a CD19 antibody or other compositions associated with CD19 antibody of the present disclosure according to the methods of the present disclosure, the subject shows observable and/or measurable reduction in or absence of one or more signs and symptoms of the particular disease. For example, for cancer, reduction in the number of cancer cells or absence of the cancer cells; reduction in the tumor size; inhibition (i.e., slow to some extent and preferably stop) of tumor metastasis; inhibition, to some extent, of tumor growth; increase in length of remission, and/or relief to some extent, one or more of the symptoms associated with the specific cancer; reduced morbidity and mortality, and improvement in quality of life issues. Reduction of the signs or symptoms of a disease may also be felt by the patient. Treatment can achieve a complete response, defined as disappearance of all signs of cancer, or a partial response, wherein the size of the tumor is decreased, preferably by more than 50 percent, more preferably by 75%. A patient is also considered treated if the patient experiences stable disease. In some embodiment, the cancer patients are still progression-free in cancer after one year, preferably after 15 months. These parameters for assessing successful treatment and improvement in the disease are readily measurable by routine procedures familiar to a physician of appropriate skill in the art.

The terms "modulating" and "altering" include "increasing" and "enhancing" as well as "decreasing" or "reducing," typically in a statistically significant or a physiologically significant amount or degree relative to control. In specific embodiments, immunological rejection associated with transplantation of the blood substitutes is decreased relative to an unmodified or differently modified stem cell by at least 10%, at least 15%, at least 20%, at least 25%, at least 30%, at least 40%, at least 50%, at least 60%, at least 70%, at least 80%, at least 90%, at least 100%, at least 150%, at least 200%, at least 300%, at least 400%, at least 500%, or at least 1000%.

An "increased" or "enhanced" amount is typically a "statistically significant" amount, and may include an increase that is 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

A "decreased" or "reduced" or "lesser" amount is typically a "statistically significant" amount, and may include a decrease that is about 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

By "obtained from" is meant that a sample such as, for example, a polynucleotide or polypeptide is isolated from, or derived from, a particular source, such as the desired organism or a specific tissue within the desired organism. "Obtained from" can also refer to the situation in which a polynucleotide or polypeptide sequence is isolated from, or derived from, a particular organism or tissue within an organism. For example, a polynucleotide sequence encoding a reference polypeptide described herein may be isolated from a variety of prokaryotic or eukaryotic organisms, or from particular tissues or cells within a certain eukaryotic organism. A "therapeutically effective amount" refers to an amount of an antibody or a drug effective to "treat" a disease or disorder in a subject. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See preceding definition of "treating."

"Chronic" administration refers to administration of the agent(s) in a continuous mode as opposed to an acute mode, so as to maintain the initial therapeutic effect (activity) for an extended period of time. "Intermittent" administration is a treatment that is not consecutively done without interruption, but rather is cyclic in nature.

"Vector" includes shuttle and expression vectors. Typically, the plasmid construct will also include an origin of replication (e.g., the ColE1 origin of replication) and a selectable marker (e.g., ampicillin or tetracycline resistance), for replication and selection, respectively, of the plasmids in bacteria. An "expression vector" refers to a vector that contains the necessary control sequences or regulatory elements for expression of the antibodies including antibody fragment of the present disclosure, in bacterial or eukaryotic cells. Suitable vectors are disclosed below.

The CD19 antigen is an antigen of about 90 kDa identified, for example, by the HD237 or B4 antibody (Kiesel et al., Leukemia Research II, 12:1119 (1987)). CD19 is found on cells throughout differentiation of B-lineage cells from the stem cell stage through terminal differentiation into plasma cells, including but not limited to, pre-B cells, B cells (including naive B cells, antigen-stimulated B cells, memory B cells, plasma cells, and B lymphocytes) and follicular dendritic cells. CD19 is also found on B cells in human fetal tissues. The term "antibody" is used in the broadest sense and specifically covers monoclonal antibodies (including full-length monoclonal antibodies), multispecific antibodies (e.g., bispecific antibodies), and antibody fragments so long as they exhibit the desired biological activity or function.

The biological activity of the CD19 binding and humanized CD19 binding antibodies of the present disclosure (not claimed) will include at least binding of the antibody to human CD19, and/or binding to human and other primate CD19 (including cynomolgus monkey, rhesus monkey, chimpanzees). Typically, the C19 binding agent binds with an affinity of at least about 1×10-7 M, and preferably 10-8 M to 10-9 M, 10-10 M, 10-11 M, or 10-12 M and binds to the predetermined antigen with an affinity that is at least two-fold greater than its affinity for binding to a non-specific antigen (e.g., BSA, casein) other than the predetermined antigen or a closely-related antigen.

"Antibody fragments" comprise a portion of a full-length antibody, generally the antigen-binding or variable region of the antibody. Examples of antibody fragments include Fab, Fab', F(ab')2, and Fv fragments; diabodies; linear antibodies; single-chain antibody molecules (e.g., scFv); and multispecific antibodies formed from antibody fragments.

"Fv" is the minimum antibody fragment which contains a complete antigen-recognition and - binding site. This fragment consists of a dimer of one heavy- and one light-chain variable region domain in tight, non-covalent association. From the folding of these two domains emanate six hypervariable loops (3 loops each from the H and L chain) that contribute the amino

acid residues for antigen binding and confer antigen binding specificity to the antibody. However, even a single variable domain (or half of a Fv comprising only three complementarity determining regions (CDRs) specific for an antigen) has the ability to recognize and bind antigen, although at a lower affinity than the entire binding site.

A monoclonal antibody as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies and is not to be construed as requiring the production of the antibody by any particular method. For example, the monoclonal antibodies to be used in accordance with the present disclosure may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

Functional fragments of the CD19 binding antibodies of the present disclosure are those fragments that retain binding to CD19 with substantially the same affinity as the intact full-length molecule from which they are derived and show biological activity including depleting B cells as measured by in vitro or in vivo assays such as those described herein.

Variable refers to the fact that certain segments of the variable domains (V domains) differ extensively in sequence among antibodies. The V domain mediates antigen binding and defines the specificity of a particular antibody for its particular antigen. However, the variability is not evenly distributed across the 10-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991)). The constant domains are not involved directly in binding an antibody to an antigen but exhibit various effector functions, such as participation of the antibody in antibody-dependent cellular cytotoxicity (ADCC).

Hypervariable region refers to the amino acid residues of an antibody which are responsible for antigen-binding. The hypervariable region generally comprises amino acid residues from a CDR (e.g. around about residues 24-34 (LI), 50-56 (L2) and 89-97 (L3) in the VL, and around about 31-35B (HI), 50-65 (H2) and 95-102 (H3) in the VH (Kabat et al., Sequences of Proteins of Immunological Interest, 5th Ed. Public Health Service, National Institutes of Health, Bethesda, Md. (1991)) and/or those residues from a "hypervariable loop" (e.g. residues 26-32 (LI), 50-52 (L2) and 91-96 (L3) in the VL, and 26-32 (HI), 52A-55 (H2) and 96-101 (H3) in the VH (Chothia and Lesk J. Mol. Biol. 196:901-917 (1987)).

Chimeric antibodies (immunoglobulins) have a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al. Proc. Natl Acad. Sci. USA 81:6851-6855 (1984)). Humanized antibody as used herein is a subset of chimeric antibodies.

Humanized forms of non-human (e.g., murine) antibodies are chimeric antibodies which contain minimal sequence derived from non-human immunoglobulin. In some embodiments, humanized antibodies are human immunoglobulins (recipient or acceptor antibody) in which hypervariable region residues of the recipient are replaced by hypervariable region residues from a non-human species (donor antibody) such as a mouse, rat, rabbit or nonhuman primate having the desired specificity, affinity, and capacity. In some embodiments, humanized antibodies are antibodies derived from human cells or from transgenic animals (typically mice) with express human antibody genes.

B cell depletion refers to a reduction in B cell levels in an animal or human after drug or antibody treatment, as compared to the B cell level before treatment. B cell levels are measurable using well-known assays such as those described in the Experimental Examples. B cell depletion can be complete or partial. As disclosed herein, but not claimed, the depletion of CD19 expressing B cells is at least 25%. Not to be limited by any one mechanism, possible mechanisms of B-cell depletion include ADCC, CDC, apoptosis, modulation of calcium flux, or a combination of two or more of the preceding.

An autoimmune disease may refer to a non-malignant disease or disorder arising from and directed against an individual's own (self) antigens and/or tissues.

The embodiments herein further find utility in a method for treating a subject having a CD 19 positive tumor. The method includes administering a modified cell to the subject having the CD19 positive tumor. In some embodiments, the modified cell may produce and/or contain an antigen recognition domain having the humanized antibody and an intracellular domain. The modified cell is a T cell. This genetically modified cell may obtain from memory cells in the subject and replicates in vivo. The modified cells are administered intravenously to the subject and persist in the subject. In some embodiments, the modified cell is an autologous T cell.

Embodiments relate to a human T cell including a nucleic acid sequence encoding a CAR. The CAR includes a CD19 antigen binding domain including the amino acid sequence of SEQ ID NO:37. In embodiments, the CAR includes a transmembrane domain, and an intracellular domain and a signaling domain of a costimulatory molecule.

In some embodiments, the intracellular domain may include a CD3-zeta signaling domain.

In embodiments, the antigen binding fragment is a scFv. The scFv includes the amino acid sequence of SEQ ID NO:37.

In some embodiments, the T cell comprises a vector that comprises the nucleic acid sequence. For example, the vector is a lentiviral vector.

In some embodiments, a pharmaceutical composition comprising the human T cell as described above.

CARs are molecules generally including an extracellular and intracellular domain. The extracellular domain includes a target-specific binding element. The intracellular domain (e.g., cytoplasmic domain) includes a costimulatory signaling region and a zeta chain portion. The costimulatory signaling region refers to a portion of the CAR including the intracellular domain of a costimulatory molecule. Costimulatory molecules are cell surface molecules other than antigens receptors or their ligands that are required for an efficient response of lymphocytes to antigen.

Between the extracellular domain and the transmembrane domain of the CAR, there may be incorporated a spacer domain. As used herein, the term "spacer domain" generally means any oligo- or polypeptide that functions to link the transmembrane domain to, either the extracellular domain or, the cytoplasmic domain of the polypeptide chain. A spacer domain may include up to 300 amino acids, preferably 10 to 100 amino acids and most preferably 25 to 50 amino acids.

In embodiments, the antigen binding element of the CAR of the disclosure targets CD19. The antigen binding element of the CAR of the disclosure includes anti-CD 19 scFV including the amino acid sequence of SEQ ID NO: 37.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

Also disclosed herein, but not claimed, are vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from oncoretroviruses such as murine leukemia viruses in that they can transduce non-proliferating cells, such as hepatocytes. They also have the added advantage of low immunogenicity.

The expression of natural or synthetic nucleic acids encoding CARs is typically achieved by operably linking a nucleic acid encoding the CAR polypeptide or portions thereof to one or more promoters, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

Additional information related to expression synthetic nucleic acids encoding CARs and gene transfer into mammalian cells is provided in U.S. Pat. No. US8,906,682.

The embodiments find utility in methods for treating a patient for an illness including administering to the patient an effective amount of the engineered cells of the present disclosure. Various illnesses can be treated according to the present methods including cancer, such as ovarian carcinoma, breast carcinoma, colon carcinoma, glioblastoma multiforme, prostate carcinoma and leukemia. In some embodiments, the method includes administering to a human patient a pharmaceutical composition including an effective antitumor amount of a population of human T cells, wherein the human T cells of the population include human T cells that comprise the nucleic acid sequence as described in the present disclosure.

Cancers that may be treated include tumors that are not vascularized, or not yet substantially vascularized, as well as vascularized tumors. The cancers may include non-solid tumors (such as hematological tumors, for example, leukemias and lymphomas) or may include solid tumors. Types of cancers to be treated with the CARs of the disclosure include, but are not limited to, carcinoma, blastoma, and sarcoma, and certain leukemia or lymphoid malignancies, benign and malignant tumors, and malignancies, e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyngioma, ependymoma, pinealoma, hemangioblastoma, acoustic neuroma, oligodendroglioma, meningioma, neuroblastoma, retinoblastoma and brain metastases).

Generally, the cells activated and expanded as described herein may be utilized in the treatment and prevention of diseases that arise in individuals who are immunocompromised. In particular, the engineered cells of the present disclosure are used in the treatment of cancer. In certain embodiments, the cells of the present disclosure are used in the treatment of patients at risk of developing cancer. Thus, the present disclosure describes, but does not claim, methods for the treatment or prevention of cancer comprising administering to a subject in need thereof, a therapeutically effective amount of the engineered T cells of the present disclosure.

The engineered T cells of the present disclosure may be administered either alone or as a pharmaceutical composition in combination with diluents and/or with other components such as IL-2 or other cytokines or cell populations. Briefly, pharmaceutical compositions of the present disclosure may include a target cell population as described herein, in combination with one or more pharmaceutically or physiologically acceptable carriers, diluents or excipients.

Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are preferably formulated for intravenous administration.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "a tumor-inhibiting effective amount", or "therapeutic amount" is indicated, the precise amount of the compositions of the present disclosure to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹cells/kg body weight, preferably 10⁵ to10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

As disclosed herein, but not claimed, it may be desired to administer activated T cells to a subject and then subsequently redraw blood (or have an apheresis performed), activate T cells therefrom according to the present disclosure, and reinfuse the patient with these activated and expanded T cells. This process can be carried out multiple times every few weeks. As disclosed herein, but not claimed, T cells can be activated from blood draws off from 10 ccs to 400 ccs. As disclosed herein, but not claimed, T cells are activated from blood draws of 20 cc, 30 ccs, 40 ccs, 50 ccs, 60 ccs, 70 ccs, 80 ccs, 90 ccs, or 100 ccs. Not to be bound by theory, using this multiple blood draw/multiple reinfusion protocols, may select out certain populations of T cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i. v.) injection, or intraperitoneally. As disclosed herein, but not claimed, the T cell compositions of the present disclosure are administered to a patient by intradermal or subcutaneous injection. As disclosed herein, but not claimed, the T cell compositions of the present disclosure are preferably administered by i.v. injection. The compositions of T cells may be injected directly into a tumor, lymph node, or site of infection.

As disclosed herein, but not claimed, cells activated and expanded using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the present disclosure may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, Cytoxan, fludarabine, cyclosporine, FK506, rapamycin, mycophenolic acid, steroids, FR901228, cytokines, and irradiation. These drugs inhibit either the calcium-dependent phosphatase calcineurin (cyclosporine and FK506) or inhibit the p70S6 kinase that is important for growth factor-induced signaling (rapamycin). (Liu et al., Cell 66:807-815, 1991; Henderson et al., Immun 73:316-321, 1991; Bierer et al., Curr. Opin. Immun 5:763-773, 1993; Isoniemi (supra)). As disclosed herein, but not claimed, the cell compositions of the present disclosure are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions of the present disclosure are administered following B-cell ablative therapy such as agents that react with CD20, e.g., Rituxan. For example, subjects may undergo standard treatment with high-dose chemotherapy followed by peripheral blood stem cell transplantation. As disclosed herein, but not claimed, following the transplant, subjects receive an infusion of the expanded immune cells of the present disclosure. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period of 1 and 30 days. The preferred daily dose is 1 to 10 mg per day although, in some instances, larger doses of up to 40 mg per day may be used (described in U.S. Pat. No. 6,120,766).

Additional information on the methods of cancer treatment using engineer T cells is provided in U.S. Pat. No. US8,906,682.

### Examples

### Recombinant human CD 19 antigen and anti-CD 19 murine monoclonal antibody (example not within the scope of the claims)

Recombinant anti-human CD19 murine antibody (FMC63) was prepared. Genes of variable domains of the heavy chain (SEQ ID NO: 1) and variable domains of light chains (SEQ ID NO: 2) were obtained and further optimized to obtain various polynucleotides encoding FMC63. Gene optimization includes eliminations of E.coli rare codons and eliminations of restriction sites used by related eukaryotic expression systems and phage display systems. Accordingly, recombinant polynucleotides (*FMC63VH*) encoding heavy chains of FMC63 (FMC63VH) and recombinant polynucleotides (*FMC63VK*) encoding light chains of FMC63 (FMC63VK) were designed and synthesized (*See* Table 1).

**Table 1**

| Name | Sequence | SEQ ID NO |
|---|---|---|
| FMC63VH | | 3 |
| FMC63VK | | 4 |
| *FMC63VH* | | 5 |
| *FMC63VK* | | 6 |

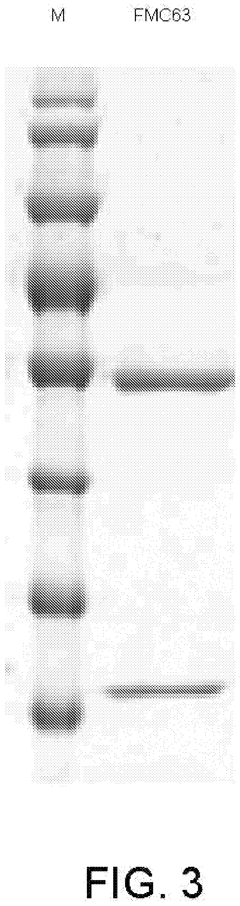

The synthesized polynucleotides encoding FMC63VH and the synthesized polynucleotides FMC63VK were cloned into two expression vectors: pMABG1 and pMABK, respectively. The plasmids expressing each of the purified heavy and light chains were co-transfected HEK293 cells. Recombinant FMC63 was expressed and obtained by using GE's Protein G affinity chromatography. Electrophoresis indicates that the purity of FMC63 is greater than 95% (FIG. 3).

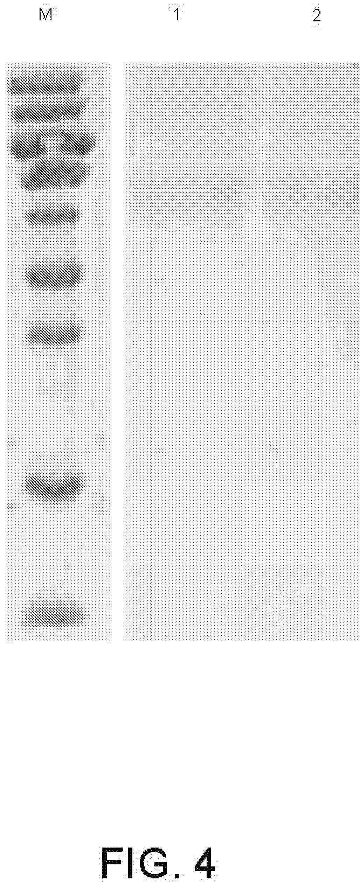

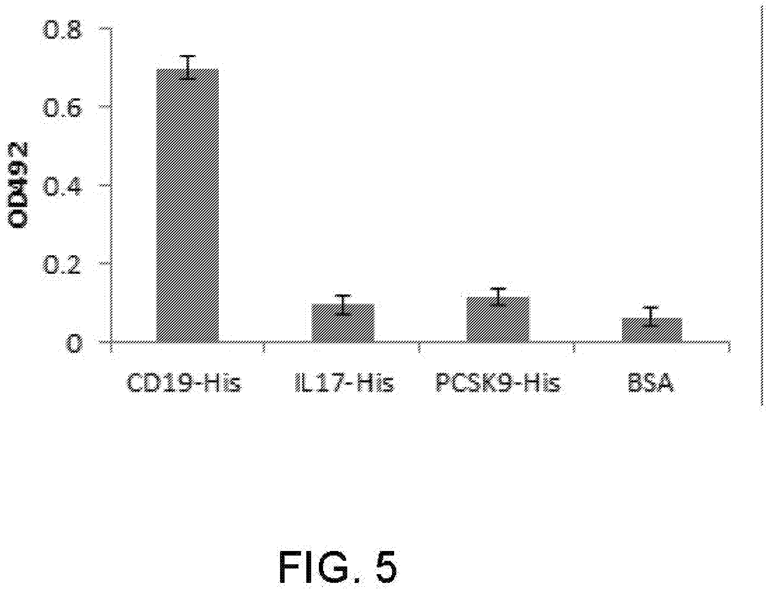

Recombinant human CD19 extracellular domain (CD19-ECD) were prepared. Polynucleotides encoding the CD19 extracellular domain was cloned into expression vector pTSE-His to construct recombinant CD19 antigen. The purified plasmids were transfected into HEK293 cells, and the expressed recombinant extracellular domain of CD19 (CD19-ECD-His) was purified using GE's Histrap FF affinity chromatography. SDS-PAGE shows a molecular weight of about 45-50KD due to five glycosylation sites that CD19-ECD has and due to glycosylation heterogeneity when CD19-ECD was expressed in HEK293 cells (FIG. 4). Recombinant murine antibody FMC63 features were verified. The binding capacity between the recombinant FMC63 and recombinant human CD19 antigen was detected using ELISA. The results show that the recombinant murine antibody is capable of specifically binding to recombinant human CD19-ECD (FIG. 5).

### Humanization of heavy chains of murine antibody FMC63 (example not within the scope of the claims)

Humanized FMC63 heavy chain variable region was designed. First, the heavy chain complementarity-determining regions (HCDRs) and framework regions (FRs) of FMC63VH were determined. According to the definition of Kabat database, sequences of the HCDRs and FRs of FMC63VH are provided in Table 2.

**Table 2**

| Name | Sequence | SEQ ID NO: |
|---|---|---|
| FMC63VH | | 3 |
| HCDR1-Kabat | GVSLPDYGVS | 7 |
| HCDR2-Kabat | VIWGSETTYYNSALKS | 8 |
| HCDR3-Kabat | KHYYYGGSYAMDY | 9 |
| FR1- Kabat | EVKLQESGPGLVAPSQSLSVTCTVS | 10 |
| FR2- Kabat | WIRQPPRKGLEWLG | 11 |
| FR3- Kabat | RLTIIKDNSKSQVFLKMNSLQTDDTAIYYCA | 12 |
| FR3- Kabat | WGQGTSVTVSS | 13 |

According to IMGT database definitions, HCDRs and FRs of FMC63VH are provided in Table 3.

**Table 3**

| Name | Sequence | SEQ ID NO: |
|---|---|---|
| FMC63VH | | 3 |
| | | |
| HCDR1-IMGT | GVSLPDYG | 14 |
| HCDR2-IMGT | IWGSETT | 15 |
| HCDR3-IMGT | KHYYYGGSYAMDY | 16 |
| FR1- IMGT | EVKLQESGPGLVAPSQSLSVTCTVS | 17 |
| FR2- IMGT | VSWIRQPPRKGLEWLGV | 18 |
| FR3- IMGT | YYNSALKSRLTIIKDNSKSQVFLKMNSLQTDD TAIYYCA | 19 |
| FR3 | WGQGTSVTVSS | 20 |

Humanized templates were selected, and searches were performed using NCBI / IGBLAST tool to search for the highest homology to human antibody genes template IGHV4-59 ^{∗} 05 based on FR1-CDR1-FR2-CDR2-FR3 sequence of FMC63VH. Searches were also performed using NCBI / IGBLAST tool to highest homology search of human antibody genes template IGHV3-48 ^{∗} 03 based on FR1-FR2-FR3 sequence of FMC63VH. Searches were also performed using NCBI / IGBLAST tool to search for homology island's tallest man J region sequence IGHJ4 ^{∗} 01 and IGHJ6 ^{∗} 01 based on FR4 sequence of FMC63VH. Sequences of the templates are provided in Table 4.

**Table 4**

| Name | Sequence | SEQ ID NO: |
|---|---|---|
| U03893\|IGHV3-48^{∗}03 | | 21 |
| M95118\|IGHV4-59^{∗}05 | | 22 |
| J00256\|IGHJ6^{∗}01 | YYYYYGMDVWGQGTTVTVSS | 23 |
| J00256\|IGHJ4^{∗}01\| Homo sapiens\|F | YFDYWGQGTLVTVSS | 24 |

Humanized HCDRs of FMC63 were designed. Based CDR grafting strategy, various templates were selected, and three different humanized FMC63VH molecules were designed accordingly. Comparison between three different humanized FMC63VH is summarized in Table 5.

**TABLE 5**

| Polynucleotide Names | HCDR definitions | FR1-FR3 template | FR4 template | SEQ ID NO: |
|---|---|---|---|---|
| hFMC63VH | IMGT | IGHV4-59^{∗}05 | IGHJ4^{∗}01 | 25 |
| h1FMC63VH | Kabat | IGHV4-59^{∗}05 | IGHJ4^{∗}01 | 26 |
| h2FMC63VH | Kabat | IGHV3-48^{∗}03 | IGHJ4^{∗}01 | 27 |

With respect to *hFMC63VH*, the HCDRs of FMC63VH were transplanted to selected frame areas of IGHV4-59 ^{∗} 05. The definition of HCDR is in accordance with the reference IMGT database, and IGHJ4 ^{∗} 01 was select for FR4. The first version polynucleotides encoding the humanized heavy chain hFMC63VH was obtained accordingly (SEQ ID NO: Annex 4-1). With respect to *h1FMC63VH*, the HCDRs of FMC63VH were transplanted to select the frame area IGHV4-59 ^{∗} 05. The definition of HCDR is in accordance with the reference Kabat database, and IGHJ4 ^{∗} 01 was selected for FR4. The second version polynucleotides encoding humanized heavy chain *h1FMC63VH* was obtained accordingly (SEQ ID NO: Annex 4-2). With respect to h2FMC63VH, the HCDRs of FMC63VH were transplanted to choose IGHV3-48 ^{∗} 03 framework regions. The definition of HCDR is in accordance with the reference Kabat HCDR database, and IGHJ6 ^{∗} 01 was selected for FR4. The third version polynucleotides encoding the humanized FMC63VH polynucleotide h1FMC63VH was obtained accordingly (SEQ ID NO: Annex A-3)

Humanized FMC63VH were cloned and then expressed. Synthesized polynucleotides of three humanized heavy chain variable regions (hFMC63VH, h1FMC63VH, h2FMC63VH) are provided in Table 6.

**TABLE 6**

| Polynucleotides | Sequences | SEQ ID NO: |
|---|---|---|
| *hFMC63VH* | | 28 |
| | | |
| *h1FMC63VH* | | 29 |
| *h2FMC63VH* | | 30 |

Then these three humanized antibody heavy chain polynucleotides were expressed in vectors: pTSEG1n-S; named pTSEG1n-hFMC63VH, pTSEG1n-h1FMC63VH, and pTSEG1n-h2FMC63VH. The heavy chain gene encoding FMC63VH was also cloned to the expression vector pTSEG1n-S including the human antibody heavy chain gene. Accordingly, the expression vector (pTSEG1n-FMC63VH) was constructed to contain the murine-human chimeric heavy chain.

The light chain genes FMC63VK of the murine donor was cloned into the expression vector pTSEK-S containing the human antibody light chain. Accordingly, the expression vector (pTSEK-FMC63VK) was constructed to contain the murine-human chimeric light chain.

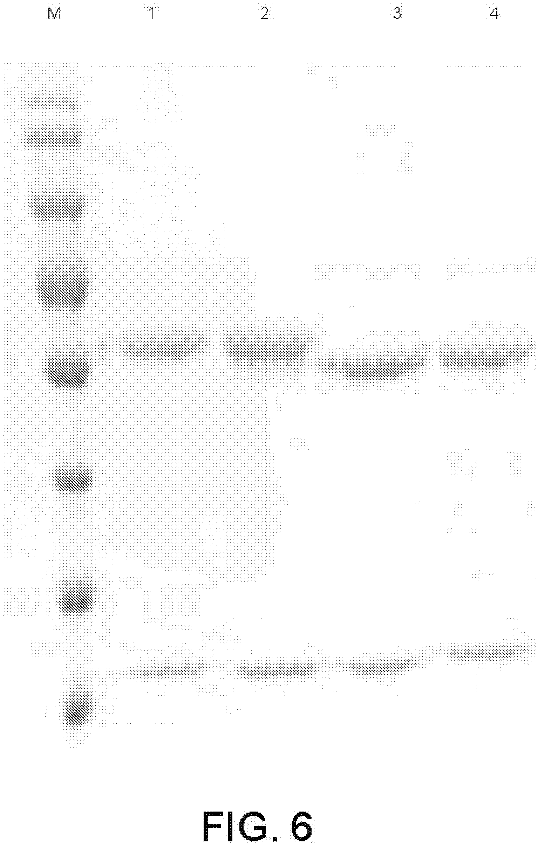

As illustrated in Table 7, the light and heavy chain expression vectors were co-transfected to HEK293 cells and were transiently expressed. After 3-4 days, supernatants were harvested. Four types of recombinant antibodies were purified using the use of Protein G affinity chromatography, and the purity thereof is more than 95% (FIG. 6).

The chimeric-FMC63 is a murine-human chimeric antibody (i.e., commonly referred to as the first generation of humanized antibody); wherein the variable regions are identical to antibodies of the murine donor while the constant region was replaced with human antibody constant region. Thus, the chimeric antibodies theoretically fully maintain biological activity of the antibodies of the murine donor. For the rest of humanized antibodies, various version of humanized heavy chains was adopted while the light chains are the same as the light chain of Chimeric FMC63 (*See* Table 7).

**TABLE 7**

| | Chimeric-FMC63 | hFMC63 | h1FMC63 | h2FMC63 |
|---|---|---|---|---|
| Heavy chain expression plasmid | pTSEG1n-FMC63VH | pTSEG1n-hFMC63VH | pTSEG1n-hlFMC 63VH | pTSEG1n-h2FMC 63VH |
| Light chain expression plasmid | pTSEK-FMC63VK | pTSEK-FMC63VK | pTSEK-FMC63VK | pTSEK-FMC63VK |

Activity comparison of heavy chains of the humanized antibody molecules was performed using Raji cells. Raji cell is a strain derived from human B lymphoma cell line. Raji cells were purchased from Chinese Academy of Medical Sciences, Institute of Basic Medical Cell Resource Center. The surface of Raji cells expresses antigen CD19, and the anti-CD19 antibody can be used to analyze the binding capacity of recombinant humanized monoclonal antibodies. By flow cytometry (IFA), binding abilities of the four recombinant humanized antibodies were analyzed. The binding analysis was performed between four humanized FMC63 and antigen CD19 expressed on Raji cell surfaces. The results show that the third version of humanized antibody (h2FMC63) binds to the antigen expressed on the surface of Raji cells. Further, the binding ability of h2FMC63 reaches 82% of chimeric FMV63. This indicates that humanized heavy chain h2FMC63VH remains the binding ability to human CD19 antigen as compared with the binding ability of chimeric FMV63 (*See* Table 8).

**TABLE 8**

| Humanized antibody | MFI (GeoMean) | Relative affinity |
|---|---|---|
| Chimeric-FMC(FMC) | 404 | 100% |
| hFMC | 67 | 17% |
| hlFMC | 186 | 46% |
| h2FMC | 330 | 82% |

### Humanization of light chains of murine antibody FMC63 (example not within the scope of the claims)

Humanized light chain of FMC63 was designed and generated by first building a light chain mutant library with storage capacity reaching 10⁸. The human-derived light chain replacement library (Fab library) was built using a dual-carrier phage display system and the humanized heavy chain gene (h2FMC63VH). The correct rate for the light chain library is above 90%.

The light chain mutant library was screened, and various clones were identified. Recombinant CD19-His antigens were prepared. Using CD19-His antigens, the light chain mutant library were screened two rounds and enriched. More than 400 clones were identified using phage-ELISA, and more than 80% of these clones were positive. Among these clones, eight clones with the highest positive values were selected for further sequence analysis. The result shows that these 18 clones have different sequences. Among these 18 different clones, four clones were Lambda light chains, while the remaining 14 clones were kappa light chains.

The 18 clones were then prepared and purified to obtain phage-Fabs. After quantitation of the purified phage-Fabs, relative affinity analysis was performed on these phage-Fab using phage-ELISA. The result shows that three clones (L2D8, L2D12, L5F3, Specific sequences of Annex 6) have the highest affinity values (Fig. 5). Sequences of these three light chains are provided in Table 9.

**TABLE 9**

| Name | Sequence | SEQ ID NO: |
|---|---|---|
| L2D8 | | 34 |
| L2D12 | | 35 |
| L5F3 | | 36 |

### Recombinant humanized murine antibody FMC63 (example not within the scope of the claims)

Three light chain gene (L2D8, L2D12, L5F3) were selected and cloned into light chain expression vectors. Using HEK293 transient expression system, three humanized monoclonal antibodies were obtained. Three kinds of people humanized antibody heavy chain gene are h2FMC63VH (pTSEG1n-h2FMC63VH).

Preliminary analysis on antibody activity was performed using cell lines expressing human CD19 CD19-K562 (3 #, 20141224) and flow cytometry (FCM). The results are illustrated in Table 10.

**TABLE 10**

| antibody Sample | FMC63VH + FMC63VK | h2FMC63VH + L5F3 | h2FMC63VH +L2D12 | h2FMC63VH + L2D8 |
|---|---|---|---|---|
| MFI(Geo) | 47.2 | 147.3 | 55.5 | 125.3 |

The above data show that three new humanized antibody (L5F3, L2D12, L2D8) shows stronger binding abilities than murine antibody FMC63, wherein L5F3 and L2D8 shows significant improvement with respect to binding ability.

### Cytotoxicity comparison between Two CARs

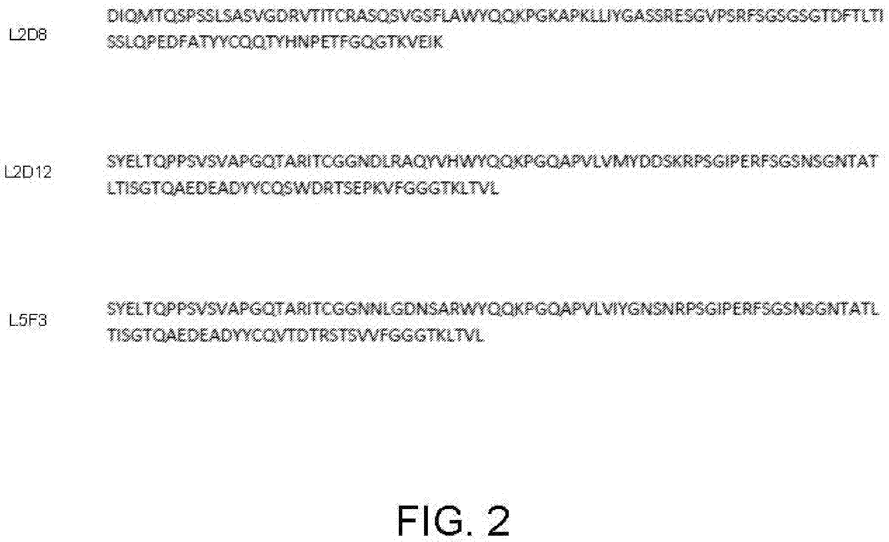

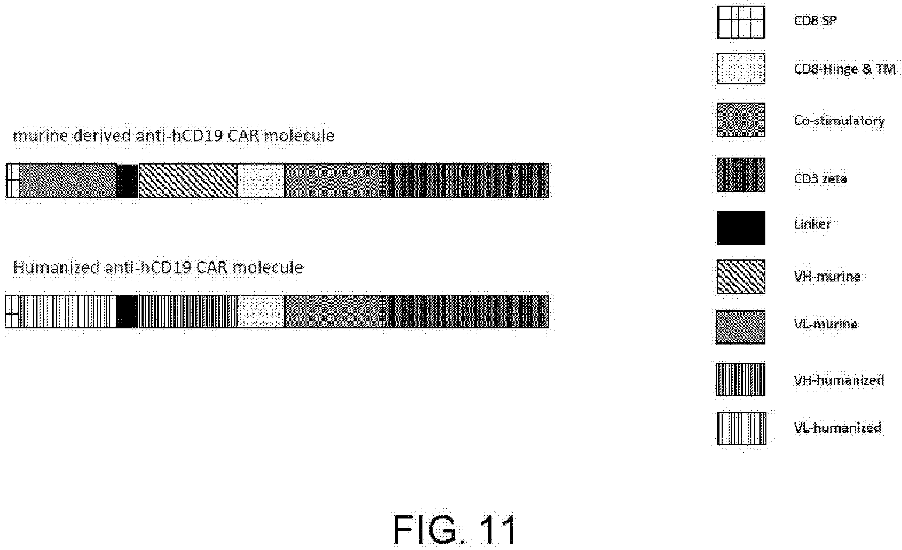

The modification was made on L2D8 such that the mouse antibody light chain CDR region took the place of the corresponding portion of the L2D8, the new sequence to obtain L2D8-2 (SEQ ID: 37). Target cells (i.e., K562-CD19 mCherry) and various effector cells (i.e., T cells expressing CARs provided in FIG. 11) or negative control cells (i.e., non-transduced T cells) were cultured for about 24 hours with various ratios between the target cells and effector cells or negative control cells. IFN-gamma production of transduced or non-transduced T cells was measured. As shown in FIGS. 2 and 3, IFN-gamma productions by T cells expressing humanized CAR was more than those by T cells expressing murine CAR and un-transduced CAR.

Techniques related to cell cultures, construction of cytotoxic T-lymphocyte assay may be found in Chimeric Receptors Containing CD137 Signal Transduction Domains Mediate Enhanced Survival of T Cells and Increased Antileukemic Efficacy in Vivo Molecular Therapy vol. 17 no. 8, 1453-1464 Aug. 2009.

**TABLE 11**

| Name | Sequence | SEQ ID NO: |
|---|---|---|
| L2D8-2 | | 37 |
| L2D8-2-DNA | | 38 |

## Claims

1. A human T cell comprising a nucleic acid sequence encoding a chimeric antigen receptor (CAR), wherein the CAR comprises a CD19 antigen binding domain comprising the amino acid sequence of SEQ ID NO:37, wherein the antigen binding fragment is a scFv and the CAR further comprises a transmembrane domain, and an intracellular domain and a signaling domain of a costimulatory molecule.

2. The human T cell of claim 1, wherein the intracellular domain comprises a CD3-zeta signaling domain.

3. The human T cell of claim 1, wherein the T cell comprises a vector that comprises the nucleic acid sequence.

4. The human T cell of claim 3, wherein the vector is a lentiviral vector.

5. A pharmaceutical composition comprising the human T cell of claim 1.

6. The human T cell of any one of claims 1 to 4, or a pharmaceutical composition of claim 5, for use in the treatment of cancer.

## Patentansprüche

1. Humane T-Zelle, umfassend eine Nukleinsäuresequenz, die einen chimären Antigenrezeptor (CAR) kodiert, wobei der CAR eine CD19-Antigen-Bindungsdomäne umfasst, die die Aminosäuresequenz von SEQ ID NO: 37 umfasst, wobei das Antigen-bindende Fragment ein scFv ist und das CAR ferner eine Transmembrandomäne und eine intrazelluläre Domäne und eine Signaldomäne eines kostimulatorischen Moleküls umfasst.

2. Humane T-Zelle nach Anspruch 1, wobei die intrazelluläre Domäne eine CD3-Zeta-Signaldomäne umfasst.

3. Humane T-Zelle nach Anspruch 1, wobei die T-Zelle einen Vektor umfasst, der die Nukleinsäuresequenz umfasst.

4. Humane T-Zelle nach Anspruch 3, wobei der Vektor ein lentiviraler Vektor ist.

5. Pharmazeutische Zusammensetzung, umfassend die menschliche T-Zelle nach Anspruch 1.

6. Humane T-Zelle nach einem der Ansprüche 1 bis 4 oder eine pharmazeutische Zusammensetzung nach Anspruch 5 zur Verwendung bei der Behandlung von Krebs.

## Revendications

1. Lymphocyte T humain comprenant une séquence d'acides nucléiques codant pour un récepteur antigénique chimérique (CAR), dans lequel le CAR comprend un domaine de liaison à l'antigène CD19 comprenant la séquence d'acides aminés de SEQ ID NO:37, dans lequel le fragment de liaison à l'antigène est un scFv et le CAR comprend en outre un domaine transmembranaire, et un domaine intracellulaire et un domaine de signalisation d'une molécule costimulatrice.

2. Lymphocyte T humain selon la revendication 1, dans lequel le domaine intracellulaire comprend un domaine de signalisation CD3-zêta.

3. Lymphocyte T humain selon la revendication 1, dans lequel le lymphocyte T comprend un vecteur qui comprend la séquence d'acides nucléiques.

4. Lymphocyte T humain selon la revendication 3, dans lequel le vecteur est un vecteur lentiviral.

5. Composition pharmaceutique comprenant le lymphocyte T humain selon la revendication 1.

6. Lymphocyte T humain selon l'une quelconque des revendications 1 à 4, ou composition pharmaceutique selon la revendication 5, pour une utilisation dans le traitement du cancer.


### SEQUENCE LISTING

<110> Shanghai SiDanSai Biotechnology CO.,LTD Wu, Zhao Liu, Zhigang Xiao, Lei Pu, Chengfei Cao,Zhiyuan
<120> HUMANIZED ANTI-CD19 ANTIBODY AND USE THEREOF WITH CHIMERIC ANTIGEN RECEPTOR
<130> SDS1.0010PCT2
<160> 38
<170> PatentIn version 3.5
<210> 1
   <211> 120
   <212> PRT
   <213> Mouse
<400> 1
   Glu Val Lys Leu Gln Glu Ser Gly Pro Gly Leu Val Ala Pro Ser Gln
   1               5                   10                  15
   Ser Leu Ser Val Thr Cys Thr Val Ser Gly Val Ser Leu Pro Asp Tyr
               20                  25                  30
   Gly Val Ser Trp Ile Arg Gln Pro Pro Arg Lys Gly Leu Glu Trp Leu
           35                  40                  45
   Gly Val Ile Trp Gly Ser Glu Thr Thr Tyr Tyr Asn Ser Ala Leu Lys
       50                  55                  60
   Ser Arg Leu Thr Ile Ile Lys Asp Asn Ser Lys Ser Gln Val Phe Leu
   65                  70                  75                  80
   Lys Met Asn Ser Leu Gln Thr Asp Asp Thr Ala Ile Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Ser Val Thr Val Ser Ser
           115                 120
<210> 2
   <211> 107
   <212> PRT
   <213> Mouse
<400> 2
   Asp Ile Gln Met Thr Gln Thr Thr Ser Ser Leu Ser Ala Ser Leu Gly
   1 5 10 15
   Asp Arg Val Thr Ile Ser Cys Arg Ala Ser Gln Asp Ile Ser Lys Tyr
   20 25 30
   Leu Asn Trp Tyr Gln Gln Lys Pro Asp Gly Thr Val Lys Leu Leu Ile
   35 40 45
   Tyr His Thr Ser Arg Leu His Ser Gly Val Pro Ser Arg Phe Ser Gly
   50 55 60
   Ser Gly Ser Gly Thr Asp Tyr Ser Leu Thr Ile Ser Asn Leu Glu Gln
   65 70 75 80
   Glu Asp Ile Ala Thr Tyr Phe Cys Gln Gln Gly Asn Thr Leu Pro Tyr
   85 90 95
   Thr Phe Gly Gly Gly Thr Lys Leu Glu Ile Thr
   100 105
<210> 3
   <211> 120
   <212> PRT
   <213> Artificial Sequence
<220>
   <223> Synthesized
<400> 3
   Glu Val Lys Leu Gln Glu Ser Gly Pro Gly Leu Val Ala Pro Ser Gln
   1 5 10 15
   Ser Leu Ser Val Thr Cys Thr Val Ser Gly Val Ser Leu Pro Asp Tyr
   20 25 30
   Gly Val Ser Trp Ile Arg Gln Pro Pro Arg Lys Gly Leu Glu Trp Leu
   35 40 45
   Gly Val Ile Trp Gly Ser Glu Thr Thr Tyr Tyr Asn Ser Ala Leu Lys
   50 55 60
   Ser Arg Leu Thr Ile Ile Lys Asp Asn Ser Lys Ser Gln Val Phe Leu
   65 70 75 80
   Lys Met Asn Ser Leu Gln Thr Asp Asp Thr Ala Ile Tyr Tyr Cys Ala
   85 90 95
   Lys His Tyr Tyr Tyr Gly Gly Ser Tyr Ala Met Asp Tyr Trp Gly Gln
               100                 105                 110
   Gly Thr Ser Val Thr Val Ser Ser
           115                 120
<210> 4
   <211> 107
   <212> PRT
   <213> Artificial Sequence
<220>
   <223> Synthesized
<400> 4
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Lys Tyr
               20                  25                  30
   Leu Asn Trp Tyr Gln Gln Lys Pro Asp Gly Thr Val Lys Leu Leu Ile
           35                  40                  45
   Tyr His Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Tyr
                   85                  90                  95
   Thr Phe Gly Gly Gly Thr Lys Leu Glu Ile Lys
               100                 105
<210> 5
   <211> 360
   <212> DNA
   <213> Artificial Sequence
<220>
   <223> Synthesized
<400> 5
   gaggtgaaac tgcaggagtc aggacctggc ctggtggcgc catcacagag cctgtccgtc 60
   acatgcactg tctcaggggt ctcattacct gactatggtg taagctggat tcgccagcct 120
   ccacgcaagg gtctggagtg gctgggagta atctggggta gtgaaaccac atactataat 180
   tcagctctca aatcccgcct gaccatcatc aaggacaact ccaagagcca agttttctta 240
   aaaatgaaca gtctgcaaac tgatgacaca gccatttact actgtgccaa acattattac 300
   tacggtggta gctatgctat ggactactgg ggtcaaggaa cctcagtcac cgtctcctca 360
<210> 6
   <211> 321
   <212> DNA
   <213> Artificial Sequence
<220>
   <223> Synthesized
<400> 6
   gacatccaga tgacacagac tacatcctcc ctgtctgcct ctctgggaga ccgcgtcacc 60
   atcagttgcc gtgcaagtca ggacattagt aaatacttaa attggtatca gcagaaacca 120
   gatggaactg ttaaactcct gatctaccat acatcacgct tacactcagg agtcccatca 180
   cggttcagtg gcagtgggtc tggaacagat tattctctca ccattagcaa cctggagcaa 240
   gaagatattg ccacttactt ttgccaacag ggtaatacgc ttccgtacac gttcggaggg 300
   gggactaagt tggaaatcaa a 321
<210> 7
   <211> 10
   <212> PRT
   <213> Artificial Sequence
<220>
   <223> Synthesized
<400> 7
   Gly Val Ser Leu Pro Asp Tyr Gly Val Ser
   1 5 10
<210> 8
   <211> 16
   <212> PRT
   <213> Artificial Sequence
<220>
   <223> Synthesized
<400> 8
   Val Ile Trp Gly Ser Glu Thr Thr Tyr Tyr Asn Ser Ala Leu Lys Ser
   1               5                   10                  15
<210> 9
   <211> 13
   <212> PRT
   <213> Artificial Sequence
<220>
   <223> Synthesized
<400> 9
   Lys His Tyr Tyr Tyr Gly Gly Ser Tyr Ala Met Asp Tyr
   1               5                   10
<210> 10
   <211> 25
   <212> PRT
   <213> Artificial Sequence
<220>
   <223> Synthesized
<400> 10
   Glu Val Lys Leu Gln Glu Ser Gly Pro Gly Leu Val Ala Pro Ser Gln
   1               5                   10                  15
   Ser Leu Ser Val Thr Cys Thr Val Ser
               20                  25
<210> 11
   <211> 14
   <212> PRT
   <213> Artificial Sequence
<220>
   <223> Synthesized
<400> 11
   Trp Ile Arg Gln Pro Pro Arg Lys Gly Leu Glu Trp Leu Gly
   1               5                   10
<210> 12
   <211> 31
   <212> PRT
   <213> Artificial Sequence
<220>
   <223> Synthesized
<400> 12
   Arg Leu Thr Ile Ile Lys Asp Asn Ser Lys Ser Gln Val Phe Leu Lys
   1               5                   10                  15
   Met Asn Ser Leu Gln Thr Asp Asp Thr Ala Ile Tyr Tyr Cys Ala
               20                  25                  30
<210> 13
   <211> 11
   <212> PRT
   <213> Artificial Sequence
<220>
   <223> Synthesized
<400> 13
   Trp Gly Gln Gly Thr Ser Val Thr Val Ser Ser
   1               5                   10
<210> 14
   <211> 8
   <212> PRT
   <213> Artificial Sequence
<220>
   <223> Synthesized
<400> 14
   Gly Val Ser Leu Pro Asp Tyr Gly
   1               5
<210> 15
   <211> 7
   <212> PRT
   <213> Artificial Sequence
<220>
   <223> Synthesized
<400> 15
   Ile Trp Gly Ser Glu Thr Thr
   1               5
<210> 16
   <211> 13
   <212> PRT
   <213> Artificial Sequence
<220>
   <223> Synthesized
<400> 16
   Lys His Tyr Tyr Tyr Gly Gly Ser Tyr Ala Met Asp Tyr
   1               5                   10
<210> 17
   <211> 25
   <212> PRT
   <213> Artificial Sequence
<220>
   <223> Synthesized
<400> 17
   Glu Val Lys Leu Gln Glu Ser Gly Pro Gly Leu Val Ala Pro Ser Gln
   1               5                   10                  15
   Ser Leu Ser Val Thr Cys Thr Val Ser
               20                  25
<210> 18
   <211> 17
   <212> PRT
   <213> Artificial Sequence
<220>
   <223> Synthesized
<400> 18
   Val Ser Trp Ile Arg Gln Pro Pro Arg Lys Gly Leu Glu Trp Leu Gly
   1               5                   10                  15
   Val
<210> 19
   <211> 39
   <212> PRT
   <213> Artificial Sequence
<220>
   <223> Synthesized
<400> 19
   Tyr Tyr Asn Ser Ala Leu Lys Ser Arg Leu Thr Ile Ile Lys Asp Asn
   1               5                   10                  15
   Ser Lys Ser Gln Val Phe Leu Lys Met Asn Ser Leu Gln Thr Asp Asp
               20                  25                  30
   Thr Ala Ile Tyr Tyr Cys Ala
           35
<210> 20
   <211> 11
   <212> PRT
   <213> Artificial Sequence
<220>
   <223> Synthesized
<400> 20
   Trp Gly Gln Gly Thr Ser Val Thr Val Ser Ser
   1               5                   10
<210> 21
   <211> 98
   <212> PRT
   <213> Artificial Sequence
<220>
   <223> Synthesized
<400> 21
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Glu Met Asn Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                      80
   Leu Gln Met Asn Ser Leu Arg Ala Glu Asp Thr Ala Val Tyr Tyr Cys
                   85                  90                  95
   Ala Arg
<210> 22
   <211> 96
   <212> PRT
   <213> Artificial Sequence
<220>
   <223> Synthesized
<400> 22
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Arg Ile Tyr Tyr Ser Gly Ser Thr Tyr Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
<210> 23
   <211> 20
   <212> PRT
   <213> Artificial Sequence
<220>
   <223> Synthesized
<400> 23
   Tyr Tyr Tyr Tyr Tyr Gly Met Asp Val Trp Gly Gln Gly Thr Thr Val
   1               5                   10                  15
   Thr Val Ser Ser
               20
<210> 24
   <211> 15
   <212> PRT
   <213> Artificial Sequence
<220>
   <223> Synthesized
<400> 24
   Tyr Phe Asp Tyr Trp Gly Gln Gly Thr Leu Val Thr Val Ser Ser
   1               5                   10                  15
<210> 25
   <211> 120
   <212> PRT
   <213> Artificial Sequence
<220>
   <223> Synthesized
<400> 25
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Trp Ser Trp Ile Arg Gln Pro Pro Gly Lys Gly Leu Glu Trp Ile
           35                  40                  45
   Gly Arg Ile Trp Gly Ser Glu Thr Thr Tyr Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser
           115                 120
<210> 26
   <211> 120
   <212> PRT
   <213> Artificial Sequence
<220>
   <223> Synthesized
<400> 26
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Asn Ser Ala Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser
           115                 120
<210> 27
   <211> 120
   <212> PRT
   <213> Artificial Sequence
<220>
   <223> Synthesized
<400> 27
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Val Ser Leu Pro Asp Tyr
               20                  25                  30
   Gly Val Ser Trp Val Arg Gln Ala Pro Gly Lys Gly Leu Glu Trp Val
           35                  40                  45
   Ser Val Ile Trp Gly Ser Glu Thr Thr Tyr Tyr Asn Ser Ala Leu Lys
       50                  55                  60
   Ser Arg Phe Thr Ile Ser Arg Asp Asn Ala Lys Asn Ser Leu Tyr Leu
   65                  70                  75                  80
   Gln Met Asn Ser Leu Arg Ala Glu Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser
           115                 120
<210> 28
   <211> 360
   <212> DNA
   <213> Artificial Sequence
<220>
   <223> Synthesized
<400> 28
   caggtgcaac tgcaggagtc gggcccagga ctggtgaagc cttcggagac cctgtccctc      60
   acctgcactg tctctggggt ctcattacca gactatggtt ggagctggat tcggcagccg     120
   ccggggaagg gactggagtg gattgggcgt atctggggta gtgaaaccac atactacaac     180
   ccgtccctca agagtcgcgt caccatttcc gtagacacgt ccaagaacca gttctccctg     240
   aagctgagct ctgtgaccgc cgcagacacg gctgtgtatt actgtgcgaa acattattac     300
   tacggtggta gctatgctat ggactactgg ggccaaggaa ccctggtcac cgtctcctca     360
<210> 29
   <211> 360
   <212> DNA
   <213> Artificial Sequence
<220>
   <223> Synthesized
<400> 29
   caggtgcaac tgcaggagtc gggcccagga ctggtgaagc cttcggagac cctgtccctc      60
   acctgcactg tctctggggt ctcattacca gactatggtg tgagctggat tcggcagccg     120
   ccggggaagg gactggagtg gattggggtt atctggggta gtgaaaccac atactacaac     180
   agcgccctca agagtcgcgt caccatttcc gtagacacgt ccaagaacca gttctccctg     240
   aagctgagct ctgtgaccgc cgcagacacg gctgtgtatt actgtgcgaa acattattac     300
   tacggtggta gctatgctat ggactactgg ggccaaggaa ccctggtcac cgtctcctca     360
<210> 30
   <211> 360
   <212> DNA
   <213> Artificial Sequence
<220>
   <223> Synthesized
<400> 30
   gaggtgcagc tggtggagtc tgggggaggc ttggtacagc ctggggggtc cctgagactc      60
   tcctgtgcag cctctggagt gtccctgcct gattatggcg tgtcctgggt ccgccaggct     120
   ccagggaagg ggctggagtg ggtttcagtg atctggggca gcgagacaac ctactacaac     180
   agcgccctga agtcccgatt caccatctcc agagacaatg ccaagaactc actgtatctg     240
   caaatgaaca gcctgagagc cgaggacacg gctgtgtatt actgtgcgaa gcactactac     300
   tacggcggca gctacgctat ggactactgg ggccaaggaa ccctggtcac cgtctcctca     360
<210> 31
   <211> 321
   <212> DNA
   <213> Artificial Sequence
<220>
   <223> Synthesized
<400> 31
   gatatccaga tgacccagag cccgagcagc ctgagcgcga gcgtgggtga tcgcgtgacc 60
   attacctgcc gcgcgagcca gagtgtcggt agcttcctgg cttggtatca gcagaaaccg 120
   ggtaaagcgc cgaaactgtt aatttatggt gccagcagcc gggagtctgg cgtgccgtcg 180
   cgttttagcg gctcgggttc gggcaccgat tttaccctga ccatctcgag cttgcagccg 240
   gaggacttcg ccacctacta ttgccagcaa acctaccata acccagagac cttcggtcag 300
   ggcaccaaag tggagatcaa a 321
<210> 32
   <211> 324
   <212> DNA
   <213> Artificial Sequence
<220>
   <223> Synthesized
<400> 32
   agctacgaac tgacccagcc gccgagcgtg tcggtggcgc cgggtcagac cgcgcgtatc 60
   acctgcggcg gcaacgacct ccgtgcccag tatgtccatt ggtatcagca gaaaccgggt 120
   caggcaccgg tgctggtgat gtacgacgat agtaagcgcc cgtctggcat cccggaacgc 180
   tttagcggct cgaattcggg caacaccgcg accctgacca ttagcggcac ccaggcggag 240
   gatgaggcgg actattactg ccagtcatgg gacaggacga gcgaacctaa ggtgtttggc 300
   ggtggcacca aactgaccgt gcta 324
<210> 33
   <211> 324
   <212> DNA
   <213> Artificial Sequence
<220>
   <223> Synthesized
<400> 33
   agctacgaac tgacccagcc gccgagcgtg tcggtggcgc cgggtcagac cgcgcgtatc 60
   acctgcggcg gcaacaacct tggtgacaac tctgctcgtt ggtatcagca gaaaccgggt 120
   caggcaccgg tgctggtgat ttacggcaac agtaatcgcc cgtctggcat cccggaacgt 180
   tttagcggct cgaattcggg caacaccgcg accctgacca ttagcggcac ccaggcggag 240
   gatgaggcgg actattactg ccaggtgacg gacacaagaa gcacatctgt ggtgtttggc 300
   ggtggcacca aactgaccgt gcta 324
<210> 34
   <211> 107
   <212> PRT
   <213> Artificial Sequence
<220>
   <223> Synthesized
<400> 34
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Val Gly Ser Phe
               20                  25                  30
   Leu Ala Trp Tyr Gln Gln Lys Pro Gly Lys Ala Pro Lys Leu Leu Ile
           35                  40                  45
   Tyr Gly Ala Ser Ser Arg Glu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Thr Tyr His Asn Pro Glu
                   85                  90                  95
   Thr Phe Gly Gln Gly Thr Lys Val Glu Ile Lys
               100                 105
<210> 35
   <211> 108
   <212> PRT
   <213> Artificial Sequence
<220>
   <223> Synthesized
<400> 35
   Ser Tyr Glu Leu Thr Gln Pro Pro Ser Val Ser Val Ala Pro Gly Gln
   1               5                   10                  15
   Thr Ala Arg Ile Thr Cys Gly Gly Asn Asp Leu Arg Ala Gln Tyr Val
               20                  25                  30
   His Trp Tyr Gln Gln Lys Pro Gly Gln Ala Pro Val Leu Val Met Tyr
           35                  40                  45
   Asp Asp Ser Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Ser Trp Asp Arg Thr Ser Glu Pro
                   85                  90                  95
   Lys Val Phe Gly Gly Gly Thr Lys Leu Thr Val Leu
               100                 105
<210> 36
   <211> 108
   <212> PRT
   <213> Artificial Sequence
<220>
   <223> Synthesized
<400> 36
   Ser Tyr Glu Leu Thr Gln Pro Pro Ser Val Ser Val Ala Pro Gly Gln
   1               5                   10                  15
   Thr Ala Arg Ile Thr Cys Gly Gly Asn Asn Leu Gly Asp Asn Ser Ala
               20                  25                  30
   Arg Trp Tyr Gln Gln Lys Pro Gly Gln Ala Pro Val Leu Val Ile Tyr
           35                  40                  45
   Gly Asn Ser Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Val Thr Asp Thr Arg Ser Thr Ser
                   85                  90                  95
   Val Val Phe Gly Gly Gly Thr Lys Leu Thr Val Leu
               100                 105
<210> 37
   <211> 107
   <212> PRT
   <213> synthesized
<400> 37
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Ser Lys Tyr
               20                  25                  30
   Leu Asn Trp Tyr Gln Gln Lys Pro Gly Lys Ala Pro Lys Leu Leu Ile
           35                  40                  45
   Tyr His Thr Ser Arg Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Asn Thr Leu Pro Tyr
                   85                  90                  95
   Thr Phe Gly Gln Gly Thr Lys Val Glu Ile Lys
   100 105
<210> 38
   <211> 321
   <212> DNA
   <213> synthesized
<400> 38
   gatatccaga tgacccagag cccgagcagc ctgagcgcga gcgtgggtga tcgcgtgacc      60
   attacctgca gggcaagtca ggacattagt aaatatttaa attggtatca gcagaaaccg     120
   ggtaaagcgc cgaaactgtt aatttatcat acatcaagat tacactcagg cgtgccgtcg     180
   cgttttagcg gctcgggttc gggcaccgat tttaccctga ccatctcgag cttgcagccg     240
   gaggacttcg ccacctacta ttgccaacag ggtaatacgc ttccgtacac gttcggtcag     300
   ggcaccaaag tggagatcaa a                                               321